# EUROPEAN PATENT APPLICATION

(11) **EP 1 985 580 A1**
(43) Date of publication of application: **29.10.2008**
(21) Application number: 07008622.8
(22) Date of filing: 27.04.2007
(51) Int. Cl.: C01B 3/38, C07C 29/151

(54) **Process for producing methanol synthesis gas**

(71) Applicant: METHANOL CASALE S.A., 6900 Lugano-Besso (CH)
(72) Inventor: Filippi, Ermanno, 6976 Castagnola (CH); Badano, Marco, 6900 Lugano (CH); Skinner, Geoffrey Frederick, Reading, Berkshire RG2 7AS (GB)
(74) Representative: Zardi, Marco

(57) **Abstract**

A process for producing methanol synthesis gas from the reforming of hydrocarbons with steam in a primary reformer (1) equipped with a plurality of externally heated catalytic tubes and then together with an oxidant gas in a secondary reformer (2) is characterized in that the reaction of said hydrocarbons with said steam in said primary reformer is performed at an operating pressure of more than 35 bar in the catalytic tubes and in that none of the hydrocarbons feed is supplied to said secondary reformer. This process allows to obtain high synthesis gas production capacities and a synthesis gas having a CO/H2 ratio close to the stoichiometric ratio for methanol production.

## Description

### Field of application

In its most general aspect, the present invention concerns the preparation of synthetic gas mixtures required for the manufacture of methanol.

In particular, the present invention concerns a process for producing methanol synthesis gas comprising hydrogen (H2) and carbon oxides (CO and CO2) obtained from the reforming of hydrocarbons.

The present invention also concerns a process for methanol production from synthesis gas obtained from the reforming of hydrocarbons.

Throughout this specification and the appended claims, the term "hydrocarbons" is used to indicate generally a raw material source of hydrogen and carbon, such as for example methane, natural gas, naphtha, GPL (liquefied petroleum gas) or refinery gas and mixtures thereof.

As known, in the field of synthesis gas production, more and more the need is felt of realizing processes which are easy to implement and allow to reach higher and higher production capacities with low operating and investment costs and low energy consumption.

### Prior art

As it is well known in the art, the production of synthesis gas for methanol or other chemicals may be performed by a steam reforming process in which desulphurized hydrocarbons are mixed with steam in a suitable ratio and the resulting mixture is admitted at a primary reformer in which most of the hydrocarbons in the feed are steam reformed (converted) into a mixture of carbon monoxide, carbon dioxide and hydrogen by passage over a suitable catalyst at moderate pressures, in the range of 15 to 25 bar, and high temperatures in the range of 850°C to 900°C.

As said conversion is endothermic, the catalyst is contained in a multiplicity of catalytic tubes which are heated externally by the heat of reaction supplied by the combustion of a gaseous fuel with air.

However, the steam reforming process suffers of the well-known drawback that the synthesis gas exiting from the primary reformer has a composition very different from the stoichiometric composition required for methanol synthesis due in particular to the low carbon/hydrogen ratio of the hydrocarbon feedstock.

In addition, the primary reformer needs to be operated at a low pressure and this results in limited production capacities of synthesis gas.

Besides the above-described conventional steam reforming process for the production of methanol synthesis gas, a so-called "combined reforming" is known in the art whereby a fraction of the hydrocarbons feedstock undergoes a steam reforming reaction as above in the primary reformer and the gas product exiting the primary reformer is combined with a remaining fraction of the hydrocarbon feedstock and supplied to a secondary reformer.

The secondary Reformer usually contains a suitable catalyst in a catalytic bed and a reaction space overlying the catalytic bed and is also supplied with a stream of oxidant gas (normally oxygen or oxygen-enriched air) in said reaction space.

The oxygen reacts in the reaction space above the catalyst bed with the combustible components of the product gas coming from the Primary Reformer, and of the hydrocarbon fraction not treated in the primary reformer, and the resulting combined product gas enters the catalyst bed at elevated temperature.

During passage down through the catalyst, the residual methane reacts endothermically with steam, resulting in a typical exit temperature of the Secondary Reformer outlet gas of around 1000°C with over 99% of the hydrocarbons feed converted to carbon oxides and hydrogen.

Although the combined reforming process allow to obtain higher production capacities, it suffers of the drawback that the hydrocarbon fraction not treated in the primary reformer has a low temperature (around 350°C) compared to that of the product gas exiting the primary reformer as the primary reformer needs to be operated at temperatures as high as 800-850°C in order to have an appropriate duty that keeps a low consumption of oxidant.

As a result, the hydrocarbon fraction not treated in the primary reformer needs to be pre-heated at a minimum temperature of about 600°C, before mixing with the product gas exiting the primary reformer, in order to perform the secondary reforming in a efficient way reducing the oxidant consumption.

This pre-heating that may be carried out through indirect heating in a suitable heat exchanger involves additional investment costs for the relative equipment.

Therefore, the costs for methanol production from synthesis gas obtained by the combined reforming process as above are increased.

On the other hand it is not convenient to treat all the hydrocarbon feed in the primary reformer, before entering the secondary reformer, because, for large capacity plants in particular, the resulting size of the primary reformer would be very large at traditional pressure levels. In addition, the temperature at the exit of the primary reformer limits the operating pressure, due to mechanical limitations of the catalytic tubes.

### Summary of the invention

The problem underlying the present invention is to provide a process for producing synthesis gas suitable for methanol production which is easy to implement and allows to obtain high production capabilities with low operating and investments costs as well as with low energy consumption.

This problem is solved by a process for producing methanol synthesis gas, the process comprising the steps of:
- feeding a gas flow comprising hydrocarbons and a gas flow comprising steam to a primary reformer equipped with a plurality of externally heated catalytic tubes,
- reacting said hydrocarbons with said steam in the catalytic tubes of said primary reformer, obtaining a product gas,
- feeding said product gas and an oxidant gas to a secondary reformer,
- subjecting said product gas to reaction with said oxidant gas and then to secondary reforming, obtaining said synthesis gas
characterized in that the reaction of said hydrocarbons with said steam in said primary reformer is performed at an operating pressure of more than 35 bar in the catalytic tubes and in that none of the hydrocarbons feed is supplied to the secondary reformer.

The term "product gas" as used herein indicates a partially reformed gas as obtained for example at the outlet of the primary reformer or in the reaction space of the secondary reformer which normally comprises carbon oxides (CO and CO2), hydrogen (H2), steam and unconverted hydrocarbons.

The term "synthesis gas" as used herein indicates a gas comprising CO and H2 which is appropriate for methanol synthesis, that is to say that said gas has a CO/H2 ratio corresponding or close to the stoichiometric composition or adjustable to said stoichiometric composition.

The expression "none of the hydrocarbons feed is supplied to the secondary reformer" as used herein means that no fraction of untreated (i.e. not yet reformed) hydrocarbons of the overall hydrocarbon feedstock is supplied to the secondary reformer for example directly or as a mixture for example with product gas exiting from the primary reformer. On the contrary, according to the invention, the secondary reformer is only supplied with product gas produced upstream it, for instance coming from the primary reformer and/or pre-reformer equipments and with oxidant gas.

The present invention is based on the finding that it is possible to have a synthesis gas appropriate for methanol production (in particular with CO/H2 ratio close to the stoichiometric ratio) and at the same time high production capacities - comparable to those achieved by the prior art combination reforming processes - by increasing the operating pressure in the catalytic tubes of the primary reformer to more than 35 bar and by avoiding to supply any portion of the hydrocarbons feed to said secondary reformer but preferably supplying all of the hydrocarbon feed to the primary reformer.

This finding is in sharp contrast with the constant teaching of the prior art according to which in order to obtain high production capacities of synthesis gas it is necessary to supply a fraction of the hydrocarbon feed to the secondary reforming while operating the primary reforming at high temperatures and moderate pressures.

Surprisingly, according to the process of the invention, the feasible increase in pressure is particularly large as it is possible for instance to substantially double the operating pressure in the catalytic tubes compared to the prior art processes (to obtain an operating pressure of 60 bar for instance) without the need of changing the design of the tubes currently used in the primary reformer.

Preferably, according to the invention, the operating pressure in the catalytic tubes of the primary reformer is in the range of 40-100 bar, most preferably in the range of 60-80 bar.

Preferably, in order to avoid a possible reduction of the tubes lifetime under the new operating pressures of the invention, it has been found useful to heat the tubes in such a way to obtain a outlet temperature not exceeding 750°C (preferably 650-750°C) for the product gas exiting said tubes.

The choice of the outlet temperature from the tubes depends on the operating pressure within the tubes and it decreases as the operating pressure increases so as to maintain a high tube lifetime.

For instance, in the case of catalytic tubes of conventional internal diameter of around 100 mm and wall thickness of 10-12 mm which are operated, according to the invention, at 60 bar pressure/750°C outlet temperature, it has been found that the tubes shows a very good lifetime of around 100,000 hours.

Furthermore, as in the combined reforming process of the prior art, the final synthesis gas is obtained by the process according to the invention with a high pressure and this allows to employ smaller equipment and piping downstream the reformers, so reducing the investment costs.

In particular, it is possible to employ smaller and less expensive equipments for compressing the final synthesis gas to the pressure required for conversion into methanol in a synthesis loop of a methanol plant. In addition, the energy required for this compression is reduced as the final synthesis gas is already obtained with a high pressure at the outlet of the reforming process. Therefore, a reduction of the energy consumption in a methanol plant employing high pressure synthesis gas obtained according to the invention can also be achieved.

In accordance with an aspect of the process according to the invention, the oxidant gas supplied to the secondary reformer consists of essentially pure oxygen.

In the present invention, the term "essentially pure oxygen" means a content of oxygen in the respective oxidant gas of at least 95%.

The use of essentially pure oxygen (instead of oxygen-enriched air for instance) allows to effectively convert the hydrocarbons (in particular methane) contained in the primary reformer outlet gas (the hydrocarbon content being increased as a result of both the increased pressure and reduced temperature in the primary reformer) in the space above the catalytic bed of the secondary reforming to produce carbon oxides and steam, so obtaining a product gas at elevated temperature.

Then, such product gas passes through the catalytic bed of the secondary reformer where endothermically reforming reaction occurs (exploiting the heat content of said product gas) which substantially completes the reforming process so achieving advantageously a overall hydrocarbon conversion yield which is fully comparable with that of the prior art reforming processes.

According to an embodiment of the present invention, the methanol synthesis gas process further comprises the steps of:
- feeding a gas flow comprising hydrocarbons and a first gas flow comprising steam to a pre-reformer and subjecting said flows to a pre-reforming treatment obtaining a first product gas,
- feeding said first product gas and a second gas flow comprising steam to a primary reformer equipped with a plurality of externally heated catalytic tubes,
- reacting the hydrocarbons of said first product gas with said steam of the second gas flow in the catalytic tubes of said primary reformer, obtaining a second product gas,
- feeding said second product gas and an oxidant gas to a secondary reformer,
- subjecting said second product gas to reaction with said oxidant gas and then to secondary reforming, thereby obtaining said synthesis gas,
characterized in that the reaction of the hydrocarbons of said first product gas with said steam of the second gas flow in said primary reformer is performed at an operating pressure of more than 35 bar in the catalytic tubes and in that none of the hydrocarbons feed is supplied to said secondary reformer.

The presence of a pre-reformer processing the hydrocarbon feed reduces the hydrocarbon content to be reformed in the primary reformer so avoiding the risk of coking in said primary reformer while obtaining at the same time high synthesis gas production capacities.

The present invention also concerns a process for producing methanol from gas synthesis comprising the steps of:
- feeding a gas flow comprising hydrocarbons and a gas flow comprising steam to a primary reformer equipped with a plurality of externally heated catalytic tubes,
- reacting said hydrocarbons with said steam in the catalytic tubes of said primary reformer, obtaining a product gas,
- feeding said product gas and an oxidant gas to a secondary reformer,
- subjecting said product gas to reaction with said oxidant gas and then to secondary reforming, obtaining said synthesis gas
- feeding said synthesis gas to a methanol synthesis loop and reacting it under conditions effective to obtain methanol,
characterized in that the reaction of said hydrocarbons with said steam in said primary reformer is performed at an operating pressure of more than 35 bar in the catalytic tubes and none of the hydrocarbons feed is supplied to said primary reformer.

According to an embodiment of the invention, the process for producing methanol from gas synthesis comprising the steps of:
- feeding a gas flow comprising hydrocarbons and a first gas flow comprising steam to a pre-reformer and subjecting said flows to a pre-reforming treatment obtaining a first product gas,
- feeding said first product gas and a second gas flow comprising steam to a primary reformer equipped with a plurality of externally heated catalytic tubes,
- reacting the hydrocarbons of said first product gas with said steam of the second gas flow in the catalytic tubes of said primary reformer, obtaining a second product gas,
- feeding said second product gas and an oxidant gas to a secondary reformer,
- subjecting said second product gas to reaction with said oxidant gas and then to secondary reforming, thereby obtaining said synthesis gas,
- feeding said synthesis gas to a methanol synthesis loop and reacting it under conditions effective to obtain methanol,
characterized in that the reaction of the hydrocarbons of said first product gas with said steam of the second gas flow in said primary reformer is performed at an operating pressure of more than 35 bar in the catalytic tubes and in that none of the hydrocarbons feed is supplied to said secondary reformer.

Preferably, the methanol synthesis process according to the invention further comprises the step of purging a gaseous flow comprising carbon oxides and hydrogen from said methanol synthesis loop.

The characteristics and advantages of the invention will further result from the following description of an embodiment thereof given by way of non limiting example with reference to the attached drawing.

### Brief description of the figures

Figure 1 shows schematically a plant for producing methanol synthesis gas according to an embodiment of the present invention, said plant being shown in fluid communication with a methanol synthesis loop of a methanol plant.
Figure 2 shows schematically a plant for producing methanol synthesis gas according to another embodiment of the present invention, said plant being shown in fluid communication with a methanol synthesis loop of a methanol plant.

### Detailed description of a preferred embodiment

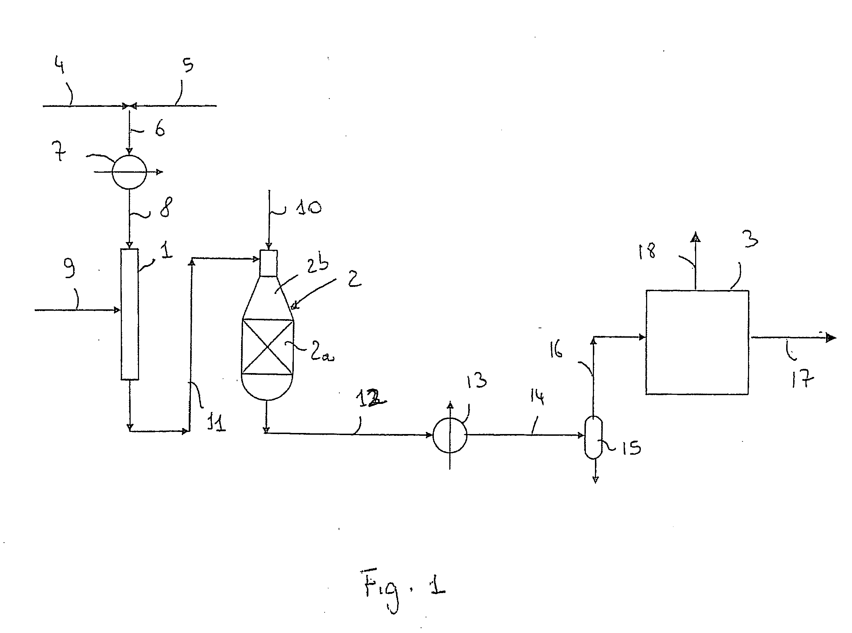

In figure 1, a plant is shown schematically that illustrates the process steps according to the present invention for producing methanol synthesis gas through primary and secondary reforming of a gaseous flow comprising hydrocarbons. In the process illustrated hereafter, the raw material employed as a source of hydrocarbons consists of natural gas.

With reference to figure 1, reference number 1 indicates a primary reformer, reference number 2 indicates a secondary reformer and block 3 indicates a methanol synthesis loop of a methanol plant respectively.

The low line 5 indicates a gas flow of natural gas which has been previously deprived of sulphur in a conventional manner and the flow line 4 indicates a flow gas of steam.

The desulphurized natural gas flow 5 is mixed with the steam flow 4 in a ratio of 1.5 to 3.5 mols of steam per mol of natural gas and the resulting mixture is fed, through the flow line 6, to a heat exchanger 7, where it is pre-heated to a temperature of around 500°C and the pre-heated mixture is fed, through the flow line 8 to the primary reformer 1.

The primary reformer 1 comprises internally a plurality of tubes (not shown) filled with a suitable catalyst, per se conventional, which are externally heated through a thermal exchange fluid indicated by the flow line 9.

According to the present invention, the catalytic tubes in the primary reformer are heated so as to have a outlet temperature from the tubes in the range of 650-750°C while the gas mixture comprising hydrocarbons and steam fed to the primary reformer 1 enters the tubes to an operating pressure of about 60 bar.

In addition, according to the invention, all the natural gas feed is supplied to the primary reformer 1.

The product gas exiting the primary reformer 1 at a temperature of 650-750°C and a pressure of around 60 bar, is supplied, through the flow line 11, to the secondary reformer 2 which also receives an oxidant flow gas consisting of essentially pure oxygen supplied through the flow line 10 in a appropriate oxidant gas/natural gas molar ratio.

The secondary reformer 2 includes, in this example, a catalytic bed 2a of a suitable catalyst (for example a nickel-based catalyst) and a overlying reaction space 2b. The oxygen fed to the secondary reformer 2 reacts with the product gas exiting the primary reformer 1 in the reaction space 2b to produce a second product gas at elevated temperature.

Then, such a second product gas passes through the catalytic bed 2a of the secondary reformer where endothermically reforming reaction occurs (exploiting the heat content of said second product gas) which substantially completes the reforming process, so obtaining a synthesis gas for methanol production.

According to an alternative embodiment (not shown), the secondary reformer 2 may be free of catalytic bed 2a. In this case, it will include one or more reaction spaces, as the reaction space 2b indicated above, for carrying out and completing the reforming process.

Advantageously, said synthesis gas is obtained with a CO/H2 ratio which is close to the stoichiometric ratio or adjustable to said stoichiometric ratio.

The final methanol synthesis gas exiting the secondary reformer 2 has a temperature of around 1000°C and a pressure of around 60 bar. It is supplied first to a plurality of heat exchangers 13 (of which only one is shown), through the flow line 12 , where it is cooled and then to a separator 15 (through the flow line 14) for the recovery of condensate.

Afterwards, the final synthesis gas is supplied to the methanol synthesis loop 3, through the flow line 16, where it is compressed to a pressure of about 80 bar and reacted in conditions effective to produce methanol. The methanol so obtained is discharged form the synthesis loop 3 through the flow line 17.

A purge flow gas comprising carbon oxides and hydrogen is also discharged from the synthesis loop 3, through the flow line 18, and recovered for other uses (such as fuel).

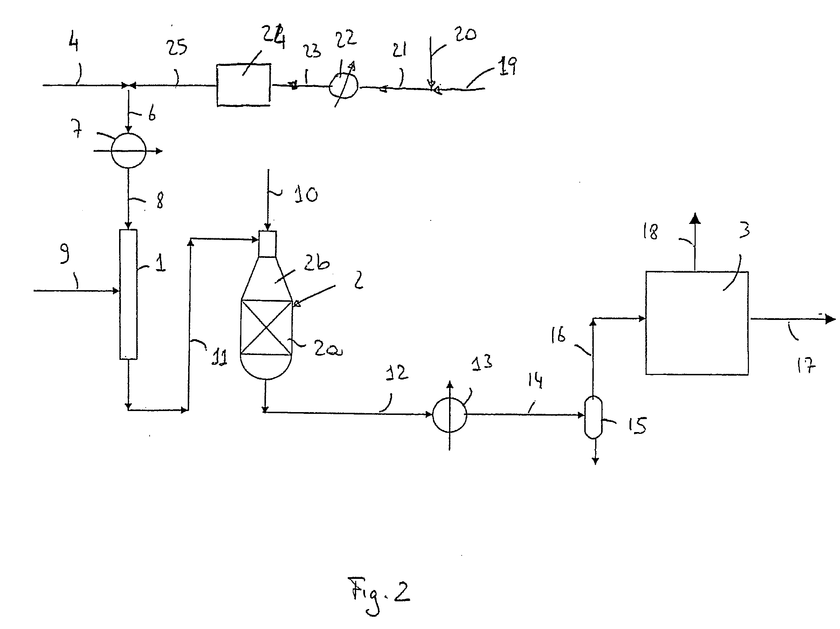

Figure 2 shows schematically a plant for producing methanol synthesis gas according to another embodiment of the present invention.

In the plant of Figure 2, elements which are structurally and/or functionally equivalent to corresponding elements of the plant of Figure 1 are indicated with the same reference numeral.

As can be seen, the plant of Figure 2 differs from that of Figure 1 in that the hydrocarbon feed (natural gas flow) is fed to a pre-reformer, herein indicated by block 24 and the resulting product gas is fed to the primary reformer 1.

More in particular, a gas flow 19 of desulphurized natural gas and a gas flow 20 of steam are mixed in an appropriate ratio and the resulting mixture is fed, through the flow line 21, to a heat exchanger 22, where it is pre-heated to an appropriate temperature and the pre-heated mixture is fed, through the flow line 23 to the pre-reformer 24.

In the pre-reformer 24, the natural gas is subjected to a pre-reforming treatment with steam, obtaining a first product gas flow 25 exiting the pre-reformer 24.

The flow 25 of first product gas exiting the pre-reformer 24 can be then mixed with additional steam coming from the flow line 4 in an appropriate ratio and the resulting gas mixture is processed in the same way as disclosed above for the plant of figure 1.

In particular, said gas mixture is pre-heated at a temperature of around 500°C in a heat exchanger 7 and fed in the primary reformer 1 at a pressure of around 60 bar.

In the primary reformer 1, the hydrocarbons of said gas mixture are subjected to reforming with steam, obtaining a second product gas exiting the primary reformer at a temperature of 650-750°C.

The second product gas is then fed to the secondary reformer 2 which also receives a flow 10 of oxidant gas and said second product gas is converted in synthesis gas for methanol production as explained above for the plant of figure 1.

Advantageously, said synthesis gas exits the secondary reformer 2 at a temperature of around 100 bar, a pressure of around 60 bar and is obtained with a CO/H2 ratio which is close to the stoichiometric ratio or adjustable to said stoichiometric ratio.

The synthesis gas is then processed, as disclosed above for the plant of figure 1, as far as the cooling in the heat exchanger 13, the separation of condensate in the separator 15, the conversion into methanol in the methanol synthesis loop 3 and the purging of a flow gas comprising carbon oxides and hydrogen from the methanol synthesis loop 3.

Of course, a man skilled in the art can bring numerous modifications and alternatives to the process according to the invention, all of which are covered by the scope of protection of the following claims.

## Claims

1. Process for producing methanol synthesis gas, the process comprising the steps of:
- feeding a gas flow comprising hydrocarbons and a gas flow comprising steam to a primary reformer (1) equipped with a plurality of externally heated catalytic tubes,
- reacting said hydrocarbons with said steam in the catalytic tubes of said primary reformer (1), obtaining a product gas,
- feeding said product gas and an oxidant gas to a secondary reformer (2),
- subjecting said product gas to reaction with said oxidant gas and then to secondary reforming, obtaining said synthesis gas
**characterized in that** the reaction of said hydrocarbons with said steam in said primary reformer (1) is performed at an operating pressure of more than 35 bar in the catalytic tubes and **in that** none of the hydrocarbons feed is supplied to said secondary reformer (2).

2. Process for producing methanol synthesis gas, the process comprising the steps of:
- feeding a gas flow comprising hydrocarbons and a first gas flow comprising steam to a pre-reformer (24) and subjecting said flows to a pre-reforming treatment obtaining a first product gas,
- feeding said first product gas and a second gas flow comprising steam to a primary reformer (1) equipped with a plurality of externally heated catalytic tubes,
- reacting the hydrocarbons of said first product gas with said steam of the second gas flow in the catalytic tubes of said primary reformer (1), obtaining a second product gas,
- feeding said second product gas and an oxidant gas to a secondary reformer (2),
- subjecting said second product gas to reaction with said oxidant gas and then to secondary reforming, thereby obtaining said synthesis gas,
**characterized in that** the reaction of the hydrocarbons of said first product gas with said steam of the second gas flow in said primary reformer (1) is performed at an operating pressure of more than 35 bar in the catalytic tubes and **in that** none of the hydrocarbons feed is supplied to said secondary reformer (2).

3. Process according to claim 1 or claim 2, **characterized in that** said operating pressure in the catalytic tubes of the primary reformer (1) is in the range of , 40-100 bar, preferably in the range of 60-80 bar.

4. Process according to anyone of the preceding claims, **characterized in that** the outlet temperature of said gas product or said second gas product from the tubes of the primary reformer (1) does not exceed 750°C.

5. Process according to claim 3, **characterized in that** said operating pressure in the catalytic tubes of the primary reformer (1) is about 60 bar and **in that** said outlet temperature is in the range of 650-750°C.

6. Process according to any of the preceding claims, **characterized in that** said oxidant gas is essentially pure oxygen.

7. Process for producing methanol from gas synthesis, the process comprising the steps of:
- feeding a gas flow comprising hydrocarbons and a gas flow comprising steam to a primary reformer (1) equipped with a plurality of externally heated catalytic tubes,
- reacting said hydrocarbons with said steam in the catalytic tubes of said primary reformer (1), obtaining a product gas,
- feeding said product gas and an oxidant gas to a secondary reformer (2),
- subjecting said product gas to reaction with said oxidant gas and then to secondary reforming, obtaining said synthesis gas,
- feeding said synthesis gas to a methanol synthesis loop (3) and reacting it under conditions effective to obtain methanol,
**characterized in that** the reaction of said hydrocarbons with said steam in said primary reformer (1) is performed at an operating pressure of more than 35 bar in the catalytic tubes and **in that** none of the hydrocarbons feed is supplied to said secondary reformer (2).

8. Process for producing methanol from gas synthesis, the process comprising the steps of:
- feeding a gas flow comprising hydrocarbons and a first gas flow comprising steam to a pre-reformer (24) and subjecting said flows to a pre-reforming treatment obtaining a first product gas,
- feeding said first product gas and a second gas flow comprising steam to a primary reformer (1) equipped with a plurality of externally heated catalytic tubes,
- reacting the hydrocarbons of said first product gas with said steam of the second gas flow in the catalytic tubes of said primary reformer (1), obtaining a second product gas,
- feeding said second product gas and an oxidant gas to a secondary reformer (2),
- subjecting said second product gas to reaction with said oxidant gas and then to secondary reforming, thereby obtaining said synthesis gas,
- feeding said synthesis gas to a methanol synthesis loop (3) and reacting it under conditions effective to obtain methanol,
**characterized in that** the reaction of the hydrocarbons of said first product gas with said steam of the second gas flow in said primary reformer (1) is performed at an operating pressure of more than 35 bar in the catalytic tubes and **in that** none of the hydrocarbons feed is supplied to said secondary reformer (2).

9. The process according to claim 7 or 8, further comprising the step of purging a gaseous flow comprising carbon oxides and hydrogen from said methanol synthesis loop (3).
